# EUROPÄISCHE PATENTSCHRIFT

(11) **EP 0 838 203 B1**
(45) Veröffentlichungstag und Bekanntmachung des Hinweises auf die Patenterteilung: **28.11.2001**
(21) Anmeldenummer: 97115386.1
(22) Anmeldetag: 05.09.1997
(51) Int. Cl.: A61F 2/20

(54) **Implantat zur Medialisation der Stimmlippen**
Implant to medialize vocal cords
Implant pour la sonorisation des cordes vocales

(30) Priorität: 27.09.1996 DE 29616828 U
(43) Veröffentlichungstag der Anmeldung: 29.04.1998
(73) Patentinhaber: Heinz Kurz GmbH Medizintechnik, 72144 Dusslingen (DE)
(72) Erfinder: Friedrich, Gerhart, Prof. Dr. med., 8036 Graz (AT)
(74) Vertreter: Möbus, Rudolf, Dipl.-Ing.

(56) Entgegenhaltungen:
- EP-A- 0 535 833
- US-A- 5 197 982
- US-A- 5 306 298

## Beschreibung

Die Erfindung betrifft ein Implantat zur Medialisation der Stimmlippen gemäß dem Oberbegriff des Anspruchs 1, wie es beispielsweise aus US-A-5 197 982 bekannt ist. Diese Implantate dienen dazu, bei einer Stimmlippenlähmung die Stimmritze zu verengen und die Stimmlippen aus ihrer Lateralstellung wieder in die Mittellinie zu bringen. Hierzu wird in den Schildknorpel eine Öffnung eingebracht, durch die das Implantat eingesetzt und dadurch die Stimmlippe medialisiert wird. Nachteilig bei den bisher hauptsächlich verwendeten Implantaten ist, daß gewisse Einwände gegen das eingesetzte Material bestehen. Diese Implantate müssen vom Chirurgen aus einem Silikonblock herausgeschnitten werden. Die Anwendung ist somit zeitraubend und die Formgebung gelingt nicht immer optimal. Außerdem ist die Fixierung der Implantate schwierig, so daß eine Verschiebung möglich ist. Eine solche Verschiebung der Implantate z. B. in das Innere des Kehlkopfes kann lebensgefährliche Folgen haben, da dadurch die Atmung verlegt wird.

Der vorliegenden Erfindung liegt die Aufgabe zugrunde, ein Implantat zu schaffen, das mit einer standardisierten Technik während der Operation unter lokaler Anästhesie des Patienten rasch und sicher eingesetzt werden kann, wobei der Grad der Medialisierung individuell einstellbar ist.

Die Aufgabe wird mit einem Implantat der eingangs genannten Art erfindungsgemäß dadurch gelöst, daß zwischen dem Mittelteil und mindestens einem der hakenförmigen Endbereiche eine Biegung des Metallstreifens angeordnet ist, die nach dem Einsetzen des Implantates zwischen dem Schildknorpel und der Stimmlippe liegt. Durch Veränderung des Radius dieser Biegung läßt sich der Anpreßdruck des Mittelteils gegen die Stimmlippe variieren. Weiter läßt sich der Anpreßdruck auch dadurch in einem kleineren Bereich variieren, daß das gesamte Implantat in seinem Abstand zur Stimmlippe verändert wird. Um diese Art der Einstellung des Anpreßdruckes des Implantates zu ermöglichen, kann mindestens einer der hakenförmigen Endbereiche mittels einer Stellschraube am Schildknorpel befestigbar sein. Zur Vermeidung von Abstoßungsreaktionen des Körpers kann das Implantat entweder aus einem körperverträglichen Metall gefertigt und/oder mit einem körperverträglichen Überzug versehen sein. Als Material für das Implantat kommen beispielsweise Titanblech oder aber auch Gold- oder Edelstahlblech in Frage.

Ein spezieller Vorteil der vorliegenden Erfindung ist, daß das Implantat nach seinem Einsetzen in den Schildknorpel sicher positioniert werden kann, so daß eine Verschiebung des Implantates, eventuell sogar in die Luftwege mit lebensbedrohlichen Komplikationen, nicht möglich ist. Dies wird durch die spezielle Formgebung des Implantates und das Umbiegen der Metallenden erreicht, und kann durch die Fixierung des Implantates mittels einer Stellschraube am Schildknorpel noch weiter abgesichert werden.

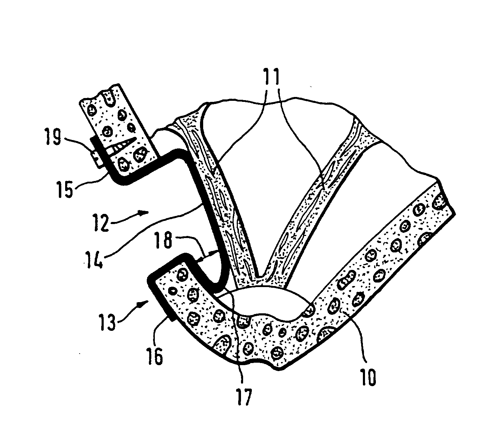

Nachfolgend wird ein bevorzugtes Ausführungsbeispiel eines erfindungsgemäßen Implantates anhand der Zeichnung näher erläutert.

Die einzige Figur zeigt einen Teilschnitt durch den menschlichen Kehlkopf. Es ist der im vorderen Kehlkopfbereich angeordnete Schildknorpel 10 zu erkennen, von den aus sich Stimmlippen 11 zu nicht näher dargestellten Stellknorpeln 20 erstrecken. In dem Schildknorpel 10 ist seitlich eine Öffnung 12 eingebracht worden, durch die ein Implantat 13 für eine der beiden Stimmlippen 11 eingesetzt ist. Das Implantat 13 ist in der Figur durch eine dicke schwarze Linie gekennzeichnet und aus einem Metallstreifen gebogen. Es weist einen Mittelteil 14 auf, der gegen die Stimmlippe 11 anliegt. Die Endbereiche 15 und 16 des Implantates 13 sind hakenförmig ausgebildet und umgreifen die Ränder der Öffnung 12 im Schildknorpel 10. Zwischen dem Mittelteil 14 und dem unteren hakenförmigen Endbereich 16 des Implantates 13 ist eine Biegung 17 des Metallstreifens angeordnet. Diese Biegung 17 liegt zwischen dem Schildknorpel 10 und der Stimmlippe 11 und erzeugt somit eine Vorspannung des Mittelteils 14 gegen die Stimmlippe 11. Der Grad der Vorspannung kann durch Veränderung des Radius der Biegung 17 variiert werden, was durch einen Doppelpfeil 18 angedeutet ist. Auch eine Verschiebung des Mittelteils 14 in Richtung auf die Stimmlippe 11 führt zu einer Erhöhung der Vorspannung des Mittelteils 14. Diese Verschiebung kann mittels einer Stellschraube 19 vorgenommen werden, mit der der Endbereich 15 des Implantates 13 mit dem Schildknorpel 10 verbunden ist. Bei dem dargestellten Implantat kann also die Vorspannung des Mittelteils 14 gegen die Stimmlippe 11 entweder durch Aufbiegen oder Zusammendrücken der Biegung 17 oder durch Verstellen der Schraube 19 während der Operation des Patienten unter lokaler Anästhesie vorgenommen werden. Entsprechend dem Ergebnis der ständig mit dem Patienten durchführbaren Stimmtests läßt sich somit eine optimale Vorspannung des Implantates 13 und somit eine optimale Unterstützung der Stimmlippe 11 erzielen.

## Patentansprüche

1. Implantat zur Medialisation der Stimmlippen, insbesondere nach Stimmlippenlähmungen, das in eine operativ einzubringende Öffnung im Schildknorpel einsetzbar ist, wobei das Implantat ein aus einem Metallstreifen gebogenes Element ist, das einen an die Stimmlippe anlegbaren Mittelteil (14) und hakenförmige Endbereiche (15, 16), die die Ränder der Schildknorpelöffnung (12) umgreifen, aufweist, **dadurch gekennzeichnet, daß** zwischen dem Mittelteil (14) und mindestens einem der hakenförmigen Endbereiche (16) eine Biegung (17) des Metallstreifens angeordnet ist, die nach dem Einsetzen des Implantates (13) zwischen dem Schildknorpel (10) und der Stimmlippe (11) liegt.

2. Implantat nach Anspruch 1, **dadurch gekennzeichnet, daß** mindestens einer der hakenförmigen Endbereiche (15, 16) mittels einer Stellschraube (19) am Schildknorpel (10) befestigbar ist.

3. Implantat nach Anspruch 1 oder 2, **dadurch gekennzeichnet**, das es aus einem körperverträglichen Material gefertigt und/oder mit einem körperverträglichen Überzug versehen ist.

4. Implantat nach einem der Ansprüche 1 bis 3, **dadurch gekennzeichnet, daß** es aus Titanblech gefertigt ist.

## Claims

1. Implant to medialise vocal cords, in particular after vocal cord paralysis, which can be inserted into a surgically-formed opening in the thyroid cartilage, the implant having a bent element made of a meta strip having a central part (14) which can be placed against the vocal cord and hooked end portions (15,16) encompassing the edges of the opening in the thyroid cartilage (12), **characterised in that** a bend (17) is arranged between central part (14) and at least one of the hooked end portiors (16) of the metal strip, which bend (17) is located between the thyroid cartilage (10) and the vocal cord (11) after insertion of the implant (13).

2. Implant according to Claim 1, **characterised in that** at least one of the hooked end portions (15,16) is fixable to the thyroid cartlage (10) by means of an adjusting screw (19).

3. Implant according to Claim 1 or 2, **characterised in that** it is made of a body-compatible material and/or is provided with a body-compatible sheath.

4. Implant according to one of Claims 1 to 3, **characterised in that** it is made of titanium sheet.

## Revendications

1. Implant pour la sonorisation des cordes vocales, en particulier après des paralysies de cordes vocales, lequel peut être mis en place dans le cartilage thyroïde dans une ouverture a réaliser par opération, l'implant étant un élément cintré à partir d'une bande métallique, qui comporte une partie médiane (14), applicable contre la corde vocale, et des zones d'extrémité en forme de crochets (15, 16) qui entourent les bords de l'ouverture de cartilage thyroïde (12), **caractérisé en ce qu'**entre la partie médiane (14) et au moins l'une des zones d'extrémité en forme de crochets (16) est disposé un coude (17) de la bande métallique, lequel se trouve entre le cartilage thyroïde (10) et la corde vocale (11) après la mise en place de l'implant (13).

2. Implant selon la revendication 1, **caractérisé en ce qu'**au moins l'une des zones d'extrémité en forme de crochets (15, 16) peut être fixée au cartilage thyroïde (10) au moyen d'une vis de serrage (19).

3. Implant selon la revendication 1 ou 2, **caractérisé en ce qu'**il est fabriqué en un matériau biocompatible et/ou muni d'un revêtement biocompatible.

4. Implant selon l'une des revendications 1 à 3, **caractérisé en ce qu'**il est fabriqué en tôle de titane.
